# EUROPÄISCHE PATENTSCHRIFT

(11) **EP 1 926 435 B1**
(45) Veröffentlichungstag und Bekanntmachung des Hinweises auf die Patenterteilung: **04.03.2009**
(21) Anmeldenummer: 06793093.3
(22) Anmeldetag: 31.08.2006
(51) Int. Cl.: A61B 6/04, G01B 11/26

(54) **PATIENTENLAGERUNGSVORRICHTUNG FÜR EINE STRAHLENTHERAPIE**
APPARATUS FOR SUPPORTING A PATIENT DURING RADIATION THERAPY
DISPOSITIF POUR POSITIONNER UN PATIENT POUR UNE RADIOTHERAPIE

(30) Priorität: 19.09.2005 DE 102005044651
(43) Veröffentlichungstag der Anmeldung: 04.06.2008
(73) Patentinhaber: SIEMENS AKTIENGESELLSCHAFT, 80333 München (DE)
(72) Erfinder: HERRMANN, Klaus, 90403 Nürnberg (DE); RIETZEL, Eike, 64289 Darmstadt (DE); SOMMER, Andres, 91094 Langensendelbach (DE)
(86) Internationale Anmeldenummer: PCT/EP2006/065851
(87) Internationale Veröffentlichungsnummer: WO 2007/033894

(56) Entgegenhaltungen:
- EP-A1- 0 687 443
- EP-A2- 0 066 272
- WO-A-03/039212
- US-A- 4 842 259
- US-A- 4 868 385
- US-A- 5 553 112
- US-A- 6 094 760
- US-B1- 6 510 615

## Beschreibung

Die Erfindung betrifft eine Patientenlagerungsvorrichtung für eine Strahlentherapie, insbesondere für eine Partikelbestrahlungseinrichtung.

Eine für eine Strahlentherapieeinrichtung vorgesehene Patientenlagerungsvorrichtung ist beispielsweise aus der DE 102 21 180 A1 bekannt. Die Patientenlagerungsvorrichtung umfasst eine Patientenliege, welche höhenverstellbar, verschiebbar, sowie um mehrere vertikale Achsen verschwenkbar ist. Um die Patientenliege exakt in der Horizontalen zu nivellieren, sind mehrere, vorzugsweise drei, Nivelliermittel, welche einen mittels einer Schraub- oder Gewindespindel verschiebbaren Keil aufweisen, vorgesehen. Der auf der nivellierten Patientenliege befindliche Patient kann unter Verwendung eines so genannten Laserkreuzes derart positioniert werden, dass der zu bestrahlende Bereich im Isozentrum der Bestrahlungseinrichtung liegt. Die Bestrahlungseinrichtung weist eine Gantry mit einem Strahlerkopf auf, welche um eine horizontale Achse schwenkbar ist.

Die US 6,094,760, als nächster Stand der Technik gesehen, offenbart ein Liegesystem für die Strahlentherapie, bei dem ein Encoder vorgesehen ist, der eine Rotation der Liege um eine Rollachse codiert.

Die US 4,868,385 offenbart eine Vorrichtung zur Detektion eines Rotationsstatus unter Verwendung mehrerer Lichtstrahlen.

Der Erfindung liegt die Aufgabe zugrunde, eine Patientenlagerungsvorrichtung für die Strahlentherapie mit gegenüber dem Stand der Technik erweiterter Funktionalität anzugeben.

Diese Aufgabe wird erfindungsgemäß gelöst durch eine Patientenlagerungsvorrichtung mit den Merkmalen des Anspruchs 1. Diese Patientenlagerungsvorrichtung weist eine Patientenliege auf, die um mindestens eine Achse schwenkbar ist. Die Achse ist vorzugsweise eine horizontale Achse, weist zumindest eine horizontale Komponente auf. Zweckdienlicherweise ist die Patientenliege hierbei um genau zwei horizontale, zueinander senkrechte Achsen schwenkbar. Um die Patientenliege gezielt in geneigter, das heißt nicht horizontaler, Anordnung zu positionieren, umfasst die Patientenlagerungsvorrichtung weiter ein Lasermesssystem. Durch die Möglichkeit, einen Patienten auf einer in definierter Weise gegenüber der Horizontalen verkippten Patientenliege zu lagern, ist die Patientenlagerungsvorrichtung besonders für Strahlentherapieeinrichtungen geeignet, bei denen die Strahlenquelle oder ein Strahlenbündel nicht oder nur in beschränktem Umfang um eine horizontale Achse schwenkbar ist. Insbesondere ist die Patientenlagerungsvorrichtung für eine Bestrahlungseinrichtung, die mit Partikelstrahlung arbeitet, geeignet.

Das Lasermesssystem umfasst hierbei vorzugsweise einen oder mehrere Laserstrahler sowie mindestens ein Anzeigemittel, insbesondere in Form eines Messlineals oder einer Messscheibe. Bevorzugt ist hierbei jeder Achse, um die die Patientenliege schwenkbar ist, zumindest ein Laserstrahler und zumindest ein Anzeigemittel zugeordnet. Ein Strahlenbündel des Laserstrahlers trifft im Betrieb des Lasermesssystems auf das Anzeigemittel auf.

Das Anzeigemittel ist derart ausgebildet, dass aufgrund des auftreffenden, beispielsweise fächerförmigen Laserstrahls ein Kippwinkel der Patientenliege gegenüber einer horizontalen Achse unmittelbar am Anzeigemittel ablesbar ist.

In einer bevorzugten Ausführungsform ist das Anzeigemittel an einer festen Struktur, beispielsweise an der Wand des Behandlungsraumes oder an einem unbeweglichen Teil der Bestrahlungseinrichtung, befestigt, während der Laserstrahler fest mit der Patientenliege verbunden ist. Dies hat den Vorteil, dass groß dimensionierte, bis zu mehreren Metern von der Patientenliege beabstandete Anzeigemittel verwendbar sind, die eine einfache und exakte Einstellung der Winkellage der Patientenliege ermöglichen. Vorzugsweise umfasst das Lasermesssystem mehrere Strahlenquellen, deren Strahlungsrichtungen zueinander orthogonal sind. In entsprechender Weise schließen auch die Ebenen, in welchen Messlineale oder sonstige Anzeigemittel angeordnet sind, vorzugsweise einen rechten Winkel miteinander ein. Das Lasermesssystem kann statt mehrerer einzelner Strahlenquellen auch einen Strahler aufweisen, der Licht emittiert, welches in verschiedene, bevorzugt zueinander orthogonale Richtungen geleitet wird.

An der Patientenliege ist in bevorzugter Ausgestaltung ein Stereotaxierahmen befestigt, welcher eine oder mehrere Strahlenquellen des Lasermesssystems trägt. Der Stereotaxierahmen hat eine feste Position relativ zum zu bestrahlenden Gewebe des Patienten. Bei der Planung der Bestrahlung wird die mittels der Anzeigemittel ablesbare korrekte Positionierung des Stereotaxierahmens ermittelt. In einer alternativen Ausführungsform, die keinen Stereotaxierahmen benötigt, sind die Laserstrahler direkt an der Patientenliege befestigt.

Nach einer vorteilhaften Weiterbildung ist das Anzeigemittel des Lasermesssystems verschiebbar oder verschwenkbar gelagert. Damit ist es möglich, den Messstab beziehungsweise die Messscheibe vor der Durchführung einer Bestrahlung derart einzustellen, dass bei der Positionierung der Patientenliege eine bestimmte, vorzugsweise bei jeder Bestrahlung identische Markierung am Anzeigemittel durch den Laserstrahler anzuleuchten ist. Auf diese Weise ist die korrekte Positionierung des Patienten wesentlich vereinfacht und das Risiko einer Fehlpositionierung minimiert.

In besonders bevorzugter Ausgestaltung ist das mindestens eine Anzeigemittel automatisch verstellbar. Aus der geplanten Positionierung der Patientenliege, insbesondere einem Kippwinkel, den die Patientenliege mit der Horizontalen einschließt, wird die Einstellung des Anzeigemittels berechnet. Der berechnete Einstellwert wird an eine Steuereinrichtung übertragen, die mit einer Antriebseinheit verbunden ist, welche das Anzeigemittel in die gewünschte Lage verfährt.

Alternativ zu der Anordnung der Laserstrahler an der Patientenliege und des Anzeigemittels im Raum ist in einer vorteilhaften Ausgestaltung das Anzeigemittel mittelbar oder unmittelbar an der Patientenliege befestigt und der Laserstrahler ist außerhalb der Patientenliege und unabhängig von deren Bewegungen angeordnet. Bei der mittelbaren Befestigung des Anzeigemittels ist dieses insbesondere am Stereotaxierahmen angeordnet. Insbesondere ist der Laserstrahler ortsfest im Raum angeordnet. Bei dieser Ausführungsform wird zweckdienlicher Weise auf bestehende im Raum angeordnete Laserstrahler zurückgegriffen.

Der Vorteil der Erfindung liegt insbesondere darin, dass durch eine direkt oder indirekt an einer Patientenliege befestigte Strahlenquelle eines Lasermesssystems in Zusammenwirkung mit einem selbsttätig verstellbaren Messstab und/oder einer ebenso automatisch verstellbaren Messscheibe auf komfortable Weise eine relativ zu einer horizontalen Ebene geneigte Einstellung der Patientenliege ermöglicht wird.

Nachfolgend werden mehrere Ausführungsbeispiele der Erfindung anhand einer Zeichnung näher erläutert. Hierin zeigen:
- FIG 1: in einem schematischen Querschnitt ausschnittsweise eine Patientenlagerungsvorrichtung mit einer kipp- baren Patientenliege und einem Lasermesssystem,
- FIG 2: eine perspektivische Ansicht einer Patientenlage- rungsvorrichtung mit einem an einer Patientenliege befestigten Stereotaxierahmen,
- FIG 3: mehrere lineare sowie ein kreisscheibenförmiges An- zeigemittel als Teile von Lasermesssystemen, und
- FIG 4: eine perspektivische Ansicht einer Patientenliege mit direkt an dieser befestigten Laserquellen.

Einander entsprechende oder gleichwirkende Teile sind in allen Figuren mit den gleichen Bezugszeichen gekennzeichnet.

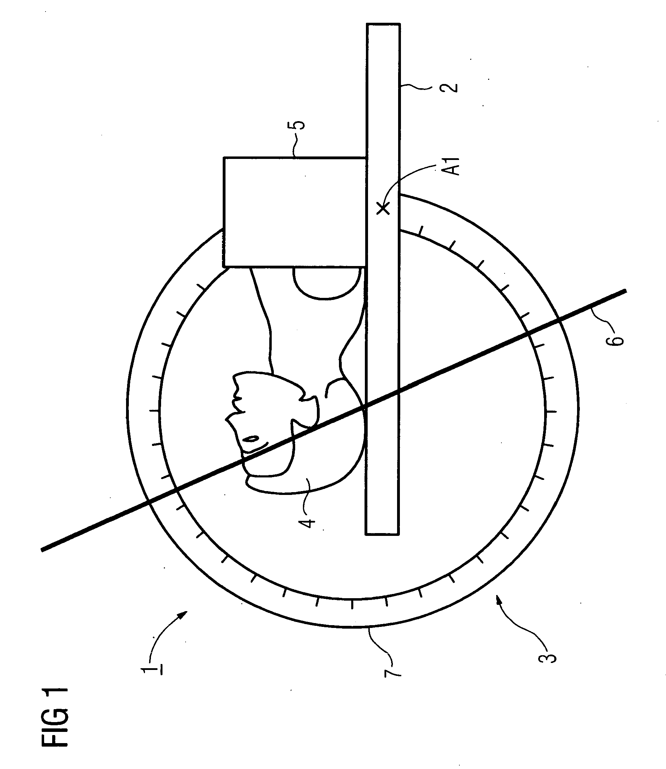

Die FIG 1 zeigt grob schematisiert eine Patientenlagerungsvorrichtung 1, welche eine Patientenliege 2 sowie ein insgesamt mit dem Bezugszeichen 3 gekennzeichnetes Lasermesssystem umfasst. Die Patientenlagerungsvorrichtung 1 ist Teil einer nicht weiter dargestellten Bestrahlungsvorrichtung, mit welcher ein Patient 4 stereotaktisch bestrahlt wird. Die den Patienten 4 tragende Patientenliege 2 ist an einer Stützkonstruktion 5 verstellbar, insbesondere um eine horizontale Achse A1 kippbar, befestigt.

Ein sichtbares Strahlenbündel 6 des Lasermesssystems 3 zeigt in Zusammenwirkung mit einer Messscheibe 7 den Kippwinkel der Patientenliege 2, das heißt denjenigen Winkel, den die Patientenliege 2 mit einer horizontalen Ebene einschließt, an. In FIG 1 sind weder Abmessungen noch Winkel maßstäblich dargestellt. Die als Anzeigemittel des Lasermesssystems 1 dienende, auch als Kreislineal bezeichnete Messscheibe 7 ist beispielsweise an einer starren Struktur der Bestrahlungsvorrichtung befestigt. In FIG 1 nicht erkennbarer Weise umfasst das Lasermesssystem 3 eine zweite Messscheibe, welche orthogonal zur sichtbaren Messscheibe 7 angeordnet ist und die Verkippung der Patientenliege 2 um eine zur Achse A1 orthogonale, ebenfalls in einer horizontalen Ebene angeordnete Achse anzeigt. Die Messscheiben 7 weisen vorzugsweise eine 360°-Einteilung auf, können jedoch auch auf sonstige Weise unterteilt sein.

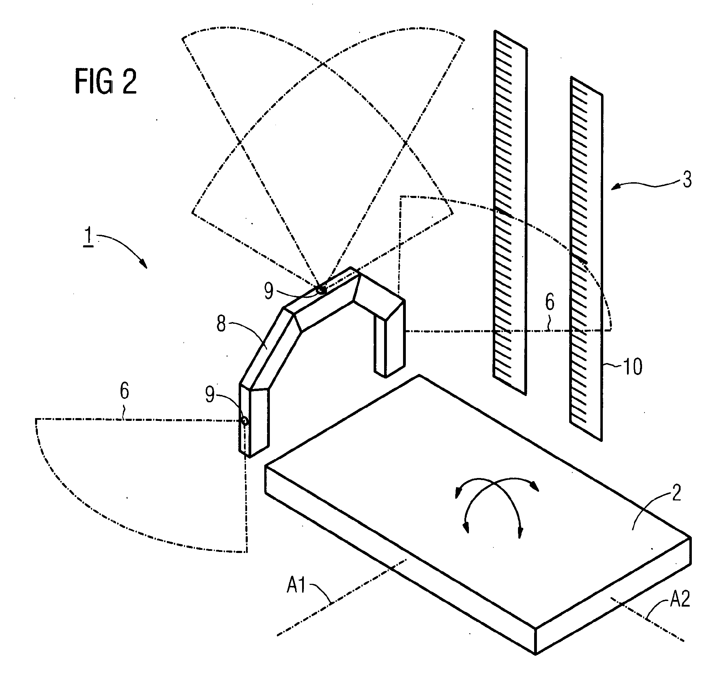

Die Vorrichtung nach FIG 2 unterscheidet sich von der Patientenlagerungsvorrichtung 1 nach FIG 1 unter anderem dadurch, dass an der Patientenliege 2 ein Stereotaxierahmen 8 befestigt ist, welcher mehrere Laserstrahler 9 trägt, deren Strahlenbündel 6 ein orthogonales System aufspannen. Die Patientenliege 2 ist sowohl um die Achse A1 als auch um eine zu dieser senkrechte, in Längsrichtung der Patientenliege 2 angeordnete Achse A2 kippbar. Eine Verschwenkung um die Achse A2 wird mittels zweier Messlineale oder Messstäbe 10 angezeigt, die an der Wand des Raumes, in welchem die Patientenlagerungsvorrichtung 1 aufgestellt ist, angebracht sind. Durch den großen Abstand der Messlineale 10 von dem zugehörigen Laserstrahler 9 ist eine hohe Auflösung des Lasermesssystems 3 gegeben. Die Messstäbe 10 dienen zugleich dazu, die Höhenpositionierung der Patientenliege 2 anzuzeigen. In nicht dargestellter Weise befindet sich an der Decke des Raumes ein weiteres Anzeigemittel 7,10, welches durch den im mittleren Bereich des Stereotaxierahmens 8 angeordneten Laserstrahler 9 angestrahlt wird.

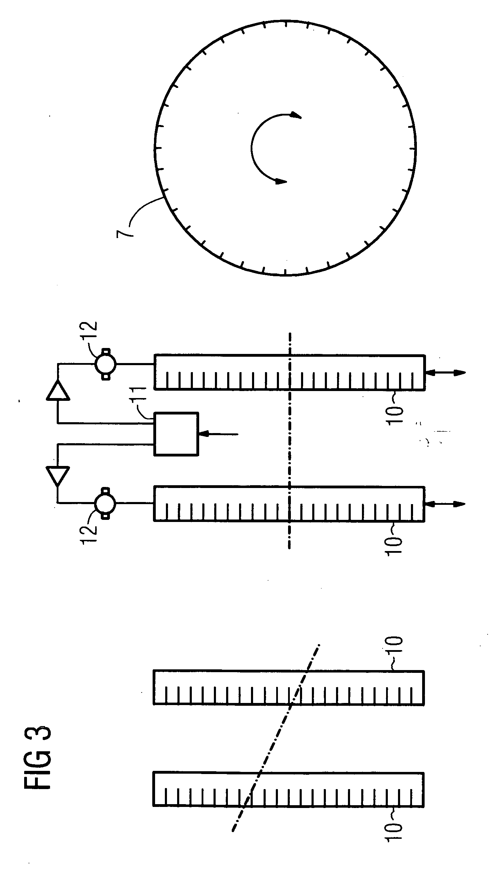

Die FIG 3 zeigt verschiedene Anzeigemittel 7,10, die im Lasermesssystem 3 verwendbar sind. Die zwei in der Darstellung linken Messlineale 10 sind fest an einer Wand oder an einer sonstigen festen Konstruktion befestigt. Die Strichmarkierungen, welche mittels des Laserstrahlers 9 erzeugt werden, sind durch eine etwa diagonal verlaufende, gestrichelte Linie angedeutet. Aus der Differenz der Höhe der Strichmarkierungen auf den beiden Messstäben 10 ist ein Kippwinkel der Patientenliege 2 berechenbar.

Bei den mittig in FIG 3 dargestellten, ebenfalls vertikal angeordneten Messlinealen 10 handelt es sich um eine Weiterentwicklung, die sich dadurch auszeichnet, dass eine motorische Höhenverstellung der Messstäbe 10 vorgesehen ist. Hierzu bietet eine zentrale Steuereinheit 11 die Möglichkeit, zwei separate Antriebseinheiten 12, nämlich eine Antriebseinheit für jeden Messstab 10, anzusteuern. An die Steuereinheit 11 wird von einer nicht dargestellten Rechenanlage, welche zur Vorbereitung der Bestrahlung genutzt wird, die Soll-Positionierung der Patientenliege 2, insbesondere einzustellende Kippwinkel, übertragen. Jeder Messstab 10 wird mittels der Steuereinheit 11 sowie der Antriebseinheiten 12 automatisch derart eingestellt, dass die Strichmarkierungen des Strahlenbündels 6 auf beiden Messstäben mit gleichen Markierungen, beispielsweise der Markierung "±0", in Übereinstimmung zu bringen ist. Die korrekte Positionierung des Patienten ist somit mit äußerst geringem Aufwand möglich.

In FIG 3 ist weiter eine Messscheibe 7 dargestellt, welche um eine normal zu dieser angeordnete Achse schwenkbar ist. Auch diese Schwenkbewegung erfolgt, analog wie die vorstehend erläuterte lineare Verstellung der Messstäbe 10, automatisch. Das von einem Laserstrahler 9 ausgestrahlte Strahlenbündel 6 erzeugt auf der Messscheibe 7 eine linienförmige Markierung, welche bei gegenüber der Horizontalen verkippter Patientenliege 2 entsprechend schräg gestellt ist. Die Messscheibe 7 wird vor der Bestrahlung mittels einer in diesem Fall nicht dargestellten Steuereinheit 11 und einer Antriebseinheit 12, beispielsweise mit einem Schrittmotor, automatisch in diejenige Winkellage gebracht, in der das Strahlenbündel 6 bei korrekt eingestellter Patientenliege 2 auf eine bestimmte Kennzeichnung, beispielsweise die "0°"-Kennzeichnung, auf der Messscheibe 7 trifft.

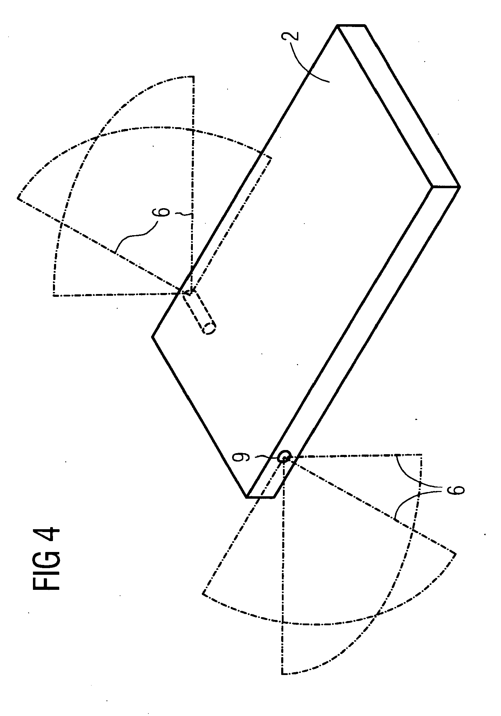

Die FIG 4 zeigt eine Patientenliege 2, die sich von der Ausführungsform nach FIG 2 hauptsächlich dadurch unterscheidet, dass die Laserstrahler 9 direkt an der Patientenliege 2 befestigt sind. Mit den Laserstrahlern 9 wirken auch im Ausführungsbeispiel nach FIG 4 automatisch verstellbare Anzeigemittel 7,10 zusammen.

## Patentansprüche

1. Patientenlagerungsvorrichtung für eine Strahlentherapie, mit zumindest einer um eine Achse (A1, A2) schwenkbaren Patientenliege (2), sowie einem Messsystem (3), welches dazu ausgebildet ist, eine bei der Bestrahlung einzustellende, nicht horizontale Ausrichtung der Patientenliege (2) anzuzeigen, **dadurch gekennzeichnet, dass** das Messsystem ein Lasermesssystem (3) ist, das zumindest einen Laserstrahler (9) sowie ein Anzeigemittel (7, 10) umfasst, auf das ein Strahlbündel (6) des Laserstrahlers (9) beim Einsatz des Lasermesssystems (3) auftrifft, und wobei das Anzeigemittel (7,10) derart ausgebildet ist, dass aufgrund des auftreffenden Strahlbündels (6) ein Kippwinkel der Patientenliege (2) gegenüber einer horizontalen Achse (A1, A2) unmittelbar vom Anzeigemittel (7,10) ablesbar ist.

2. Patientenlagerungsvorrichtung nach einem der vorhergehenden Ansprüche,
**dadurch gekennzeichnet, dass** die zumindest eine Achse eine horizontale Achse (A1, A2) ist.

3. Patientenlagerungsvorrichtung nach einem der vorhergehenden Ansprüche,
**dadurch gekennzeichnet, dass** das Lasermesssystem (3) einen Laserstrahler (9) umfasst, welcher starr mit der Patientenliege (2) verbunden ist.

4. Patientenlagerungsvorrichtung nach einem der vorhergehenden Ansprüche,
**dadurch gekennzeichnet, dass** die Patientenliege (2) mit einem Stereotaxierahmen (8) verbunden ist.

5. Patientenlagerungsvorrichtung nach Anspruch 4,
**dadurch gekennzeichnet, dass** an dem Stereotaxierahmen (8) mindestens ein Laserstrahler (9) angeordnet ist.

6. Patientenlagerungsvorrichtung nach einem der Ansprüche 3 bis 5,
**dadurch gekennzeichnet, dass** mit der Patientenliege (2) mehrere Laserstrahler (9) verbunden sind, welche orthogonal zueinander ausgerichtet sind.

7. Patientenlagerungsvorrichtung nach einem der vorhergehenden Ansprüche,
**dadurch gekennzeichnet, dass** das Lasermesssystem (3) einen Messstab (10) als Anzeigemittel umfasst.

8. Patientenlagerungsvorrichtung nach Anspruch 7,
**dadurch gekennzeichnet, dass** der Messstab (10) linear verstellbar ist.

9. Patientenlagerungsvorrichtung nach einem der vorhergehenden Ansprüche,
**dadurch gekennzeichnet, dass** das Lasermesssystem (3) eine Messscheibe (7) als Anzeigemittel umfasst.

10. Patientenlagerungsvorrichtung nach Anspruch 9,
**dadurch gekennzeichnet, dass** die Messscheibe (7) verschwenkbar gelagert ist.

11. Patientenlagerungsvorrichtung nach einem der Ansprüche 1 bis 10,
**dadurch gekennzeichnet, dass** das Anzeigemittel (7,10) automatisch verstellbar ist, wobei die Einstellung des Anzeigemittels (7,10) von der Soll-Positionierung der Patientenliege (2) abhängig ist.

12. Patientenlagerungsvorrichtung nach einem der Ansprüche 1 bis 2 und 7 bis 11, bei der das Anzeigemittel (7,10) an der Patientenliege (2) angebracht ist und der Laserstrahler (9) außerhalb der Patientenliege (2) angeordnet ist.

## Claims

1. Apparatus for supporting a patient during radiation therapy, having at least one patient couch (2) which can be pivoted about an axis (A1, A2), as well as a measuring system (3) which is embodied to this end to display a non-horizontal alignment of the patient couch (2) which is to be adjusted during the radiation process, **characterized in that** the measuring system is a laser measuring system (3), that has at least one laser emitter (9) as well as a display means (7, 10), which is struck by a beam bundle (6) of the laser emitter (9) when the laser measuring system is used, and with the display means (7, 10) being embodied such that as a result of the beam bundle (6) striking it, a tilting angle of the patient couch (2) in relation to a horizontal axis (A1, A2) can be read directly from the display means (7, 10).

2. Apparatus for supporting a patient according to one of the preceding claims, **characterised in that** the at least one axis is a horizontal axis (A1, A2).

3. Apparatus for supporting a patient according to one of the preceding claims, **characterised in that** the laser measuring system (3) includes a laser emitter (9) which is rigidly connected to the patient couch (2).

4. Apparatus for supporting a patient according to one of the preceding claims, **characterised in that** the patient couch (2) is connected to a steriotaxic frame (8).

5. Apparatus for supporting a patient according to claim 4, **characterized in that** at least one laser emitter (9) is arranged on the stereotaxic frame (8).

6. Apparatus for supporting a patient according to one of claims 3 to 5, **characterized in that** several laser emitters (9) are connected to the patient couch (2), which are aligned orthogonally to one another.

7. Apparatus for supporting a patient according to one of the preceding claims, **characterized in that** the laser measuring system (3) includes a measuring stick (10) as a display means.

8. Apparatus for supporting a patient according to claim 7, **characterized in that** the measuring stick (10) can be adjusted linearly.

9. Apparatus for supporting a patient according to one of the preceding claims, **characterized in that** the laser measuring system (3) includes a measuring disk (7) as a display means.

10. Apparatus for supporting a patient as claimed in claim 9, **characterized in that** the measuring disk (7) is mounted so as to be able to pivot.

11. Apparatus for supporting a patient as claimed in one of claims 1 to 10, **characterized in that** the display means (7, 10) can be adjusted automatically, with the adjustment of the display means (7, 10) depending on the desired positioning of the patient couch (2).

12. Apparatus for supporting a patient as claimed in one of claims 1 to 2, and 7 to 11, in which the display means (7, 10) is attached to the patient couch (2) and the laser emitter (9) is arranged outside of the patient couch (2).

## Revendications

1. Dispositif pour positionner un patient pour une radiothérapie, comportant au moins une civière de patient (2), laquelle peut pivoter autour d'un axe (A1, A2), ainsi qu'un système de mesure (3) qui est réalisé pour indiquer une orientation de la civière de patient (2) non horizontale à ajuster lors de l'irradiation, **caractérisé en ce que** le système de mesure est un système de mesure laser (3) qui comprend au moins un émetteur laser (9) ainsi qu'un moyen d'indication (7, 10) que vient frapper un faisceau de rayons (6) de l'émetteur laser (9) lors de la mise en oeuvre du système de mesure laser (3) et le moyen d'indication (7, 10) étant réalisé de manière telle qu'il est possible de lire directement sur le moyen d'indication (7, 10) un angle d'inclinaison de la civière de patient (2) par rapport à un axe horizontal (A1, A2) sur base du faisceau de rayons incidents (6).

2. Système pour positionner un patient selon l'une des revendications précédentes, **caractérisé en ce que** l'au moins un axe est un axe horizontal (A1, A2).

3. Système pour positionner un patient selon l'une des revendications précédentes, **caractérisé en ce que** le système de mesure laser (3) comprend un émetteur laser (9) qui est relié de manière fixe à la civière de patient (2).

4. Système pour positionner un patient selon l'une des revendications précédentes, **caractérisé en ce que** la civière de patient (2) est reliée à un cadre de stéréotaxie (8).

5. Système pour positionner un patient selon la revendication 4, **caractérisé en ce qu'**au moins un émetteur laser (9) est situé sur le cadre de stéréotaxie (8).

6. Système pour positionner un patient selon l'une des revendications 3 à 5, **caractérisé en ce que** sont reliés, à la civière de patient (2), plusieurs émetteurs laser (9) qui sont orientés orthogonalement entre eux.

7. Système pour positionner un patient selon l'une des revendications précédentes, **caractérisé en ce que** le système de mesure laser (3) comprend une règle (10) en tant que moyen d'indication.

8. Système pour positionner un patient selon la revendication 7, **caractérisé en ce que** la règle (10) est déplaçable linéairement.

9. Système pour positionner un patient selon l'une des revendications précédentes, **caractérisé en ce que** le système de mesure laser (3) comprend un disque de étalon (7) en tant que moyen d'indication.

10. Système pour positionner un patient selon la revendication 9, **caractérisé en ce que** le disque de étalon (7) est logé de manière à pouvoir pivoter.

11. Système pour positionner un patient selon l'une des revendications 1 à 10, **caractérisé en ce que** le moyen d'indication (7, 10) est ajustable automatiquement, le réglage du moyen d'indication (7, 10) étant fonction du positionnement de consigne de la civière de patient (2).

12. Système pour positionner un patient selon l'une des revendications 1 à 2 et 7 à 11, dans lequel le moyen d'indication (7, 10) est monté sur la civière de patient (2) et l'émetteur laser (9) se trouve en dehors de la civière de patient (2).
